# EUROPEAN PATENT APPLICATION

(11) **EP 4 763 041 A1**
(43) Date of publication of application: **24.06.2026**
(21) Application number: 25222852.3
(22) Date of filing: 12.12.2025
(51) Int. Cl.: A61B 1/00

(54) **IMAGING DEVICE, IMAGING SYSTEM, AND METHOD FOR OPERATING AN IMAGING SYSTEM**

(30) Priority: 20.12.2024 DE 102024139197
(71) Applicant: Karl Storz SE & Co. KG, 78532 Tuttlingen (DE)
(72) Inventor: Tischler, Jan-Heiko, 78532 Tuttlingen (DE); Bauer, Franz, 78532 Tuttlingen (DE)

(57) **Abstract**

The invention relates to an imaging device (10; 110), in particular an endoscope device, for stereoscopic image generation, wherein the imaging device (10; 110) has a shaft (12; 112) that defines a longitudinal axis (14; 114) and at least one optical viewing direction unit (58, 158) for generating a viewing angle (18) of greater than 0° with respect to the longitudinal axis (14; 114).

It is proposed that the imaging device (10; 110) has a periscope unit (20; 120) for combining at least two beam paths (22, 24; 122, 124) for use in stereoscopic image generation, wherein the viewing direction unit (58, 158) is rotationally fixed with respect to the shaft (12; 112) and the periscope unit (20; 120) is arranged so as to be rotatable relative to the shaft (12; 112) about the longitudinal axis (14; 114).

## Description

The present invention relates to an imaging device for stereoscopic image generation, having a shaft that defines a longitudinal axis, and at least one viewing direction unit for generating a viewing angle greater than 0° with respect to the longitudinal axis. Furthermore, the present invention relates to an imaging system having such an imaging device and a method for operating such an imaging device.

The development of modern 3D endoscopes places high demands on the precise alignment of their optical systems. From the prior art, endoscopes are known that use two spatially separated optical systems for stereoscopic image generation in order to capture an object point from different viewing angles. With these endoscopes having two optical systems, the two optical systems must always be aligned in a horizontal position to realistically replicate human depth perception. In addition to endoscopes with two optical systems, stereo endoscopes with a single optical relay system are also known from the prior art, for example US 9,883,788 B2.

However, endoscopes with an oblique viewing direction (DOV ≠ 0°) present a particular challenge: When the endoscope shaft is rotated, as is common during medical procedures to change the viewing direction, the alignment of the two optical systems changes. This leads to faulty stereoscopy and impairs the quality of 3D imaging. This problem significantly limits the application possibilities of oblique-viewing 3D endoscopes.

One approach known, for example, from US 9,798,131 B2, involves changing the viewing direction of the endoscope by rotating a distal prism, while the two optical systems remain in their horizontal alignment. Also known from EP 3 367 155 B1 is a device for capturing a stereo image with two optical systems, in which a change in the viewing direction is coupled with a translational movement of a lens by means of a cam mechanism. However, the use of two separate optical systems in 3D endoscopes presents a challenge in terms of space requirements and the complexity of the setup. Each optical system requires its own optical elements, light guidance and alignment, which results in a larger and more technically complex construction overall. This is particularly problematic in medical applications where the available space in the distal region of the endoscope is limited. This makes miniaturization more difficult, and at the same time increases the susceptibility to manufacturing defects and mechanical instabilities, which can impair reliability and functionality.

The object of the present invention is to provide an imaging device having a particularly compact structure and simplified construction, which at least partially solves the aforementioned problems according to the prior art and advantageously ensures a three-dimensional representation in different viewing directions of the imaging device.

This object is achieved, according to the invention, by a device having the features of claim 1. Advantageous configurations and developments of the invention can be found in the dependent claims.

According to the invention, an imaging device, in particular an endoscope device, is provided for stereoscopic image generation. The imaging device has a shaft that defines a longitudinal axis and at least one optical viewing direction unit for generating a viewing angle greater than 0° with respect to the longitudinal axis. The imaging device also features a periscope unit for combining at least two beam paths for use in stereoscopic image generation. The viewing direction unit is rotationally fixed with respect to the shaft, and the periscope unit is arranged so as to be rotatable relative to the shaft about the longitudinal axis.

Due to the use of a periscope unit that is rotatable relative to the shaft and, in particular, the viewing direction unit, rotation of the shaft to change the viewing direction with respect to the alignment of the two beam paths for stereoscopic image generation can be compensated. Thus, expedient stereoscopic image generation can be made possible at any rotation angle of the shaft about the longitudinal axis, i.e., in particular at different viewing angles. Due to the combination of the two beam paths, it is not necessary to use two separate optical systems. The use of only one optical system is sufficient for expedient stereoscopic image generation at different rotation angles of the shaft by the imaging device according to the invention. Thus, it is possible to provide an imaging device that exhibits a particularly simple and space-saving design. This helps to minimize a number of manufacturing and assembly-related error sources.

The imaging device can be designed as an endoscope device, which can be designed as part of an endoscope or can comprise the entire endoscope. Alternatively, the imaging device can also be designed as an exoscope device, in particular as part of an exoscope, or comprise the entire exoscope. The imaging device is designed for stereoscopic image generation, for example as a stereoendoscope or stereoexoscope. The imaging device can be configured to capture images of an object point from different perspectives in order to make stereoscopic image generation possible. The imaging device can be intended for medical or non-medical applications.

The viewing direction unit can be arranged at a distal end of the shaft. An arrangement of the viewing direction unit at the distal end of the shaft can correspond to an arrangement in a distal end region of the shaft. In other words, the viewing direction unit does not have to form the distal tip of the shaft. Distal to the viewing direction unit, a further optical element, such as a lens optic, a protective glass or the like, can be arranged. The viewing direction unit can have one or more reflective flat or curved surfaces, for example inside or on a prism or between a plurality of prisms. The two aforementioned beam paths can exit from a proximal side of the viewing direction unit.

The periscope unit can be arranged on a proximal side with respect to the viewing direction unit. The periscope unit can be configured to combine the two beam paths, in particular those located distally with respect to the periscope unit, into a common beam path, in particular into a beam path that is proximal with respect to the periscope unit. In other words, the periscope unit can be configured to combine the two beam paths, which are spaced apart from one another at its optical input, into the common beam path at its optical output.

Within the scope of the present invention, it can be provided that the imaging device has only an optical system, which can be configured to transmit light of the combined beam path emanating proximally from the periscope unit in the direction of a proximal end of the imaging device, preferably in the direction of a sensor unit. The optical system can be designed as a rod lens system or another relay lens system. Alternatively, the imaging device can also be designed without an optical system for transmitting light of the combined beam path from the periscope unit in the direction of the proximal end of the imaging device. In connection with explaining the invention, a "beam path" is to be described as a path that light beams take in an optical setup.

The periscope unit can be configured to deflect at least one of the two beam paths, preferably at least twice, in order to combine the two beam paths. In order to deflect beam paths, the periscope unit can have at least one deflection element, preferably at least two deflection elements. At least one deflection element can be designed as a reflection element, for example as a mirror element, a dispersion element or the like. For example, at least one of the two beam paths can be deflected by means of the periscope unit in a first deflection into a direction angled to the longitudinal axis, in particular substantially perpendicular to it, and in a second deflection into a direction at least approximately parallel to the longitudinal axis. The beam path combined by the periscope unit can substantially run parallel to the longitudinal axis. Preferably, the combined beam path can run at least along the longitudinal axis at least in portions.

Within the scope of the present disclosure, a proximal portion or proximal side is a portion or side that is arranged closer to the observer/user and further away from the field of view/patient than a distal portion or distal side. Similarly, a distal portion or distal side is a portion or side that is arranged closer to the field of view/patient and further away from the observer/user than a proximal portion or proximal side. Accordingly, distal can also be described as close to the patient, facing the patient, and/or distant from the observer. Proximal can also be described as distant from the patient, facing away from the patient, and/or close to the observer. When used as an endoscopic instrument, the distal end of the shaft is usually inserted into the body in order to allow observations to be made there. At least the proximal end of the instrument protrudes from the body because this is where the operator handles and controls it.

The two beam paths at the optical input of the periscope unit can be arranged in a manner spaced apart from one another. Via the two beam paths that are distal to and spaced apart with respect to the periscope unit, an object point can be viewed and, in particular, captured from two different perspectives. A first image can be captured and/or generated via a first beam path of the two beam paths, a second image can be captured and/or generated via a second beam path of the two beam paths, wherein the first image and the second image can be combined for stereoscopic image generation.

The viewing direction unit and/or the periscope unit can be arranged in the shaft. The periscope unit can be mounted in the shaft so as to be rotatable about the longitudinal axis. What is meant by the viewing direction unit being arranged in a rotationally fixed manner with respect to the longitudinal axis, within the scope of the present explanation of the invention, is that it is rotationally fixed at least with respect to rotations of the shaft about the longitudinal axis. Within the scope of the present invention, the imaging device can be designed in such a way that the viewing angle between the viewing direction and the longitudinal axis is either unchangeable or changeable.

According to a further development of the invention, the imaging device can have a movement unit that is configured to keep the periscope unit in a horizontal position when the shaft is rotated about the longitudinal axis. Thus, it can be achieved that the quality of the stereoscopic image generation can be maintained when the viewing direction is changed by rotating the shaft about the longitudinal axis. In a preferred exemplary embodiment of the invention, the movement unit can be configured to rotate the periscope unit about the longitudinal axis by an angle whose magnitude corresponds to a rotation angle of the rotation of the shaft, in particular of the viewing direction unit, about the longitudinal axis and, in particular, is opposite to this rotation angle. What is meant by the periscope unit being held in a horizontal position, in connection with the explanation of the invention, is that a parallax between the two beam paths, in particular those distal with respect to and spaced apart from the periscope unit, runs horizontally. Thus, the stereoscopic image generation can advantageously be ensured even when the shaft is rotated and the viewing direction is thereby changed.

The movement unit can, for example, have active and/or passive movement mechanisms. For example, the movement unit can have a drive, in particular a hydraulic, electric or pneumatic drive, in particular a rotary actuator, for rotating the periscope unit about the longitudinal axis. A passive movement mechanism can, for example, be a movement mechanism based on spring tension, gravity, elasticity or the like. The movement unit can have a position sensor system for determining the rotation angle of the shaft, in particular the viewing direction unit, about the longitudinal axis. The movement unit can have a control unit that is configured to control the movement mechanism as a function of the rotation angle of the shaft, as captured in particular by means of the position sensor system, in particular of the viewing direction unit, with respect to the longitudinal axis. The movement unit can be arranged at least partially inside the shaft, for example on an inner wall of the shaft. It can be provided that the periscope unit is rotatable about the longitudinal axis via the movement unit. Alternatively, the imaging device can have a mounting for the periscope unit that is designed separately from the movement unit. Via the movement unit, the periscope unit can be driven to rotate relative to the shaft.

Within the scope of the invention, it can be provided that the periscope unit provides at least two distinguishable optical channels at its optical output. Preferably, the beam path combined by the periscope unit can have the two distinguishable optical channels. The optical output of the periscope unit can be arranged on a proximal side of the periscope unit. In connection with the explanation of the invention, an "optical channel" is to be understood to mean light beams that propagate along a specific beam path, for example one of the two beam paths already mentioned and/or the combined beam path, and are able to transport image information with respect to an object.

A first optical channel of the two optical channels can be provided via a first beam path of the two beam paths, and a second optical channel of the two optical channels can be provided via a second beam path of the two beam paths. Via the optical channels for stereoscopic image generation, the two images of an object point from two different perspectives can be transmitted. For example, the first optical channel can transmit the first image and the second optical channel can transmit the second image. Advantageously, the images for stereoscopic image generation can be captured and transmitted in a horizontal position of the stereoscopic unit, in particular the two beam paths, even when the shaft is rotated about the longitudinal axis. This makes it possible to achieve particularly high-quality stereoscopic image generation even when the viewing direction changes, while at the same time maintaining a particularly compact setup of the imaging device.

According to a further development of the invention, it can be provided that the periscope unit has at least one filter, in particular the one already mentioned, in order to specify a beam characteristic with respect to at least one of the optical channels. This makes it particularly easy and reliable to distinguish between the optical channels. The filter can be arranged, for example, in the first beam path or in the second beam path in order to specify the beam characteristic of the first optical channel or the second optical channel, respectively. In a preferred exemplary embodiment, the periscope unit can have two filters in order to specify a beam characteristic of the two optical channels. For example, a first filter can be arranged in the first beam path in order to specify the beam characteristic of the first optical channel. A second filter can, for example, be arranged in the second beam path in order to specify the beam characteristic of the second optical channel.

Distinguishable optical channels can exhibit different physical properties from one another, in particular apart from information required for image generation. For example, the distinguishable optical channels exhibit different wavelengths and/or different polarizations. According to a preferred exemplary embodiment of the invention, the optical channels can form different color channels from one another, i.e., in particular, exhibit different wavelengths or wavelength ranges. For example, the first optical channel can be a blue-red color channel and the second optical channel a green color channel. Due to the different color channels, the optical channels can be distinguished on the combined beam path, so that images from different viewing angles can be transmitted via the combined beam path for stereoscopic image generation. Alternatively or additionally, the optical channels can form different polarization channels. In other words, the optical channels can be distinguished from one another by their polarization. Furthermore, the distinguishability can alternatively be achieved by at least one shutter aperture arranged on the two beam paths, wherein the capture of the images for stereoscopic image generation can be carried out by the shutter aperture as a function of a shutter status of the two beam paths. For example, a first image can be captured from a first viewing angle with a second beam path blocked by the shutter aperture, and a second image can be captured from a second viewing angle with a first beam path blocked by the shutter aperture.

According to a further development of the invention, the at least one filter can be designed as a dichroic beam splitter. Alternatively, at least one filter can be designed as a color filter or as a polarization splitter. Thus, the radiation characteristic can be specified with particular precision. Such filters can simultaneously be used as deflection elements for the beam paths. Dichroic beam splitters can be used with particular flexibility due to their transmission and reflection properties.

Within the scope of the invention, it can be provided that at least one filter is designed to be transparent to one of the two optical channels. For example, the filter can be configured to deflect the first optical channel, in particular to reflect it, and to transmit the second optical channel. According to one exemplary embodiment of the invention, it can be provided that the periscope unit deflects only one of the two beam paths. For example, the first beam path, which preferably provides the first optical channel, can run in a manner spaced apart from the longitudinal axis, and the second beam path, which preferably provides the second optical channel, and in particular the combined beam path, can run on the longitudinal axis. Therefore, a particularly simple setup of the periscope unit is sufficient in order to make stereoscopic image generation possible. This reduces both the number of possible sources of error regarding the precision of the optical setup of the imaging device and its costs.

According to a further development of the invention, the imaging device can have a sensor unit for capturing the two optical channels. The sensor unit can be connected to a computing unit of the imaging system in terms of data technology. The computing unit can be configured to process the images captured by means of the sensor unit for stereoscopic image generation. According to one exemplary embodiment, the sensor unit can have a single image sensor. The image sensor can be configured to capture the combined beam path. The image sensor can be configured to resolve the combined beam path according to the optical channels.

Thus, a single image sensor may be sufficient to capture the images for stereoscopic image generation. For example, the image sensor can be designed as an RGB sensor in order to capture a green color channel and a red-blue color channel. The image sensor can have a Bayer filter. The image sensor can, for example, be arranged in such a way that the longitudinal axis intersects the image sensor.

According to one embodiment of the invention, the imaging device can have a beam splitter positioned upstream of the sensor unit, which divides the combined beam paths according to their distinguishable optical channels. The beam splitter can, for example, be designed as a dichroic beam splitter. For example, the beam splitter can be configured to deflect at least one of the two optical channels. For example, the beam splitter can be designed in such a way that it is transparent to the first optical channel and deflects the second optical channel. The sensor unit can have a dedicated image sensor in each case for the beam paths divided according to distinguishable optical channels. The image sensors can be designed as CCD sensors, CMOS sensors or the like. Advantageously, particularly simple image sensors can be used to capture the two optical channels, in particular the two images.

The invention further relates to an imaging system having the imaging device according to the invention as described above. The imaging system can be designed as an endoscopy system or as an exoscopy system. Thus, an imaging system can be provided which reliably makes stereoscopic image generation possible even when the viewing direction changes. Due to the use of the imaging device according to the invention in the imaging system, a particularly compact system can be provided. The imaging system can have a display. The imaging system can have a computing unit for combining the images transmitted via the two beam paths and captured by means of the sensor unit for stereoscopic image generation. The computing unit can comprise at least one processor and one memory element, along with an operating program stored on the memory element. The memory element can be designed as a digital storage medium, for example as a memory chip or the like. The computing unit can be part of the imaging device or designed separately from the imaging device. The imaging device can be connected to the display via the computing unit. The computing unit can be configured to generate a stereoscopic image for outputting on the display. The display can be designed as a monitor or the like. The computing unit can be connected to the control unit of the movement unit in terms of data technology and/or control technology. For example, the computing unit and the control unit can be at least partially designed as a single unit, i.e., they can have common components.

Furthermore, the invention relates to a method for operating an imaging device for stereoscopic image generation, in particular of the type described above. In a method step, in particular in a capture step, images from different perspectives can be transmitted to the sensor unit via the two beam paths and the two distinguishable optical channels. For this purpose, the two beam paths can be combined into a single beam path by means of the periscope unit. By means of the computing unit, the images captured by the sensor unit can be combined to generate a stereoscopic image and, in particular, can be output via the display.

In a method step, in particular in a rotation step, the viewing direction of the imaging device can be changed by rotating the shaft, in particular the viewing direction unit, about the longitudinal axis. The viewing direction can be defined by the viewing angle and a rotation angle of the shaft, in particular the viewing direction unit, about the longitudinal axis. According to the method according to the invention, the periscope unit is rotated relative to the shaft when the shaft is rotated about the longitudinal axis. Within the scope of the invention, it can be provided that the periscope unit is rotated in such a way that a plane of two beam paths passing through the periscope unit, in particular the two beam paths mentioned above, is aligned at least approximately horizontally. After the periscope unit has been aligned, another capture step can be carried out. It can be provided that the computing unit is configured to rotate the combined stereoscopic image before outputting it to the display, for example as a function of the rotation angle of the shaft, and in particular to compensate for the rotation of the shaft. Due to such a method according to the invention, stereoscopic image generation can be reliably carried out even when the viewing direction changes. The method according to the invention makes a particularly compact configuration of the imaging device possible.

The devices and methods disclosed herein are not to be limited to the application and embodiment described above. In particular, they can have a number of individual elements, components and units as well as method steps, which differ from a number mentioned herein, in order to fulfill a function described herein.

It is in particular pointed out that all features and properties described with regard to a device, but also procedures, can be analogously transferred to methods in a corresponding manner and can also be used as method steps within the meaning of the invention and are considered to be disclosed as such. Likewise, method steps disclosed within the scope of the present invention description are to be regarded as device features that can be used in a device. This means that structural features mentioned in relation to methods, i.e., features relating to the device, can also be taken into account, claimed and also counted as part of the disclosure within the scope of the device claims.

The present invention will be described by way of example below with reference to the accompanying figures. The figures, the description, and the claims contain numerous features in combination. A person skilled in the art will also, expediently, consider the features individually and use them in combination as appropriate in the context of the claims.

If there is more than one example of any of the components described below, only one of them can be provided with a reference sign in the figures and in the description. The description of this example can be transferred accordingly to the other examples of the component. If objects are named using number words, such as first, second, third object, etc., these are used to name and/or assign objects. Accordingly, for example, a first object and a third object may be included, but not a second object. However, a number and/or sequence of objects could also be derived using number words.

In the drawings:
Fig. 1 shows a schematic representation of an imaging system having a display, a computing unit and an imaging device,
Fig. 2 shows a schematic representation of an optical setup of the imaging device from Fig. 1,
Fig. 3 shows a schematic representation of a periscope unit of the imaging device from Fig. 1 and 2 in a distal front view having the arrangement of two beam paths, which are spaced apart from one another, through the periscope unit,
Fig. 4 shows a schematic sequence of a method for operating the imaging device from Fig. 1 and 2,
Fig. 5 shows a schematic representation of an optical setup of an imaging device in an alternative configuration, and
Fig. 6 shows a schematic representation of a periscope unit of the imaging device from Fig. 5 in a distal front view having the arrangement of two beam paths, which are spaced apart from one another, through the periscope unit.

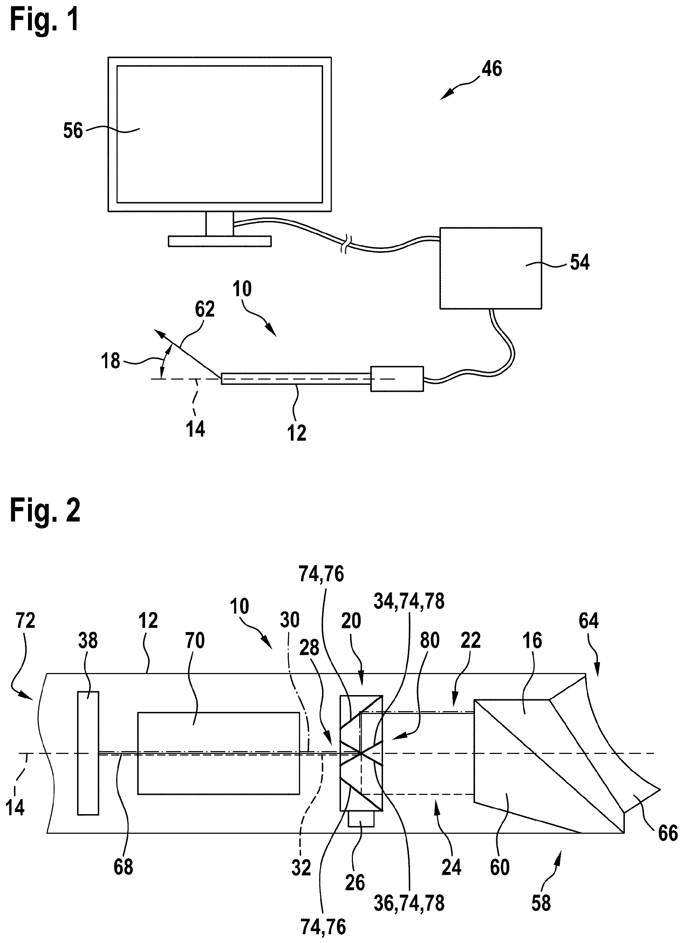

Fig. 1 is an overall representation of an imaging system 46 having an imaging device 10 for stereoscopic image generation, having a computing unit 54 and having a display 56, which is designed as a monitor.

The imaging device 10 is designed as an endoscope, in particular as a stereo endoscope. The imaging device 10 has a shaft 12 that defines a longitudinal axis 14.

The imaging device 10 is configured to capture images of an object point from different perspectives in order to make stereoscopic image generation possible. The computing unit 54 is configured to combine the images captured by means of the imaging device 10 from different perspectives for stereoscopic image generation. The computing unit 54 has a processor (not shown here) and a memory element (not shown here) along with an operating program stored on the memory element. The memory element is designed as a digital storage medium, for example as a memory chip or the like. The computing unit 54 is designed separately from the imaging device 10. Alternatively, the computing unit 54 can also be part of the imaging device 10. The display 56 is connected to the computing unit 54 in terms of data technology. The computing unit 54 is configured to generate a stereoscopic image for outputting on the display 56.

With reference to Fig. 2, the imaging device 10 has at least one optical viewing direction unit 58 for generating a viewing angle greater than 0° with respect to the longitudinal axis 14. The viewing direction unit 58 has two prisms 16, 60. Alternatively, the viewing direction unit 58 can also have only one prism 16, 60, more than two prisms 16, 60 or other deflecting elements for generating the viewing angle of greater than 0° with respect to the longitudinal axis 14. The viewing direction unit 58 is arranged at a distal end 64 of the shaft 12. Distal to the viewing direction unit 58, a plano-concave lens 66 is arranged, in particular for achieving a desired imaging. Light from an object to be imaged enters the viewing direction unit 58 from a distal side of the viewing direction unit 58. Two beam paths 22, 24 exit from a proximal side of the viewing direction unit 58. The beam path in the viewing direction unit 58 and on a distal side of the viewing direction unit 58 is not shown.

The imaging device 10 has a periscope unit 20 for combining the two beam paths 22, 24 for use in stereoscopic image generation. The periscope unit 20 is arranged on a proximal side with respect to the viewing direction unit 58. The periscope unit 20 is configured to combine the two beam paths 22, 24 into a common beam path 68, in particular into a beam path that is proximal with respect to the periscope unit 20. The two beam paths 22, 24 are spaced apart from one another at an optical input 80. The periscope unit 20 is configured to combine the two beam paths 22, 24 into a common beam path 68 at its optical output 28.

Via the two distal and spaced beam paths 22, 24 with respect to the periscope unit 20, an object point can be viewed and captured from two different perspectives. Via a first beam path 22 of the two beam paths 22, 24, a first image can be captured and generated, and via a second beam path 24 of the two beam paths 22, 24, a second image can be captured and generated, wherein the first image and the second image can be combined for stereoscopic image generation.

The periscope unit 20 is configured to combine the two beam paths 22, 24 by deflecting the two beam paths 22, 24. The periscope unit 20 is configured to deflect the two beam paths 22, 24 at two points in each case. The periscope unit 20 is configured, in a first deflection, to deflect the two beam paths 22, 24 into a direction that runs perpendicular to the longitudinal axis 14. By means of the periscope unit 20, the two beam paths 22, 24 can be deflected in a second deflection into a direction parallel to the longitudinal axis 14. The periscope unit 20 has a plurality of deflection elements 74 for deflecting the beam paths 22, 24. For the first deflection, each beam path 22, 24 is assigned a deflection element 74 designed as a mirror 76. For the second deflection, each beam path 22, 24 is assigned a deflection element 74 designed as a dichroic beam splitter 78.

The imaging device 10 has only one optical system 70 for transmitting the combined beam path 68 emanating from the periscope unit 20 in the direction of a proximal end 72 of the imaging device 10. The optical system 70 is configured to transmit the combined beam path 68 to a sensor unit 38 of the imaging device 10. The optical system 70 is designed as a rod lens system or another relay lens system. Alternatively, the imaging device 10 can also be designed without an optical system 70.

The viewing direction unit 58 is arranged in a rotationally fixed manner with respect to the shaft 12. The periscope unit 20 is arranged so as to be rotatable about the longitudinal axis 14 relative to the shaft 12. The viewing direction unit 58 and the periscope unit 20 are arranged in the shaft 12.

The imaging device 10 has a movement unit 26 that is configured to keep the periscope unit 20 in a horizontal position when the shaft 12 is rotated about the longitudinal axis 14. The movement unit 26 is configured to rotate the periscope unit 20 about the longitudinal axis 14 by an angle whose magnitude corresponds to a rotation angle of the rotation of the shaft 12, in particular of the viewing direction unit 58, about the longitudinal axis 14 and which is opposite to this rotation angle.

The movement unit 26 has a rotation actuator (not shown here) for rotating the periscope unit 20 about the longitudinal axis 14. The rotary actuator is designed, for example, as an electric rotary actuator. Alternatively, the rotary actuator can also be hydraulically or pneumatically driven, or can be based on a passive movement mechanism. The movement unit 26 has a position sensor system (not shown here) for determining the rotation angle of the shaft 12, in particular the viewing direction unit 58, about the longitudinal axis 14. The movement unit 26 has a control unit (not shown here) that is configured to control the rotation actuator as a function of the rotation angle of the shaft 12, as captured in particular by means of the position sensor system, in particular of the viewing direction unit 58, with respect to the longitudinal axis 14. The movement unit 26 is arranged in the shaft 12. The movement unit 26 is connected to the shaft 12 and serves as a mounting for the periscope unit 20.

The periscope unit 20 provides two distinguishable optical channels 30, 32 at its optical output 28. The combined beam path 68 has the two distinguishable optical channels 30, 32. The optical output 28 is arranged on a proximal side of the periscope unit 20.

A first optical channel 30 of the two optical channels 30, 32 is provided via a first beam path 22 of the two beam paths 22, 24 and a second optical channel 32 of the two optical channels 30, 32 is provided via a second beam path 24 of the two beam paths 22, 24. Via the optical channels 30, 32 for stereoscopic image generation, the two images of an object point from two different perspectives can be transmitted. For example, the first optical channel 30 can transmit the first image and the second optical channel 32 can transmit the second image.

The periscope unit 20 has two filters 34, 36 in order to specify a beam characteristic with respect to the two optical channels 30, 32. The filters 34, 36 are designed as dichroic beam splitters 78. The filters 34, 36 are formed by the deflection elements 74 designed as dichroic beam splitters 78. A first filter 34 is arranged in the first beam path 22 in order to specify the beam characteristic of the first optical channel 30. A second filter 36 is arranged in the second beam path 24 in order to specify the beam characteristic of the second optical channel 32.

Due to filters 34 and 36, optical channels 30 and 32 have different color channels. Alternatively or additionally, the optical channels 30, 32 can have different polarization channels. The first optical channel 30 is a blue-red color channel and the second optical channel 32 is a green color channel. Due to the different color channels, the optical channels 30, 32 can be distinguished on the combined beam path 68, so that images from different viewing angles can be transmitted via the combined beam path 68 for stereoscopic image generation. Alternatively or additionally, the optical channels 30, 32 can have different polarization channels.

The imaging device 10 has the sensor unit 38 for capturing the two optical channels 30, 32. The sensor unit 38 has a single image sensor. The image sensor is configured to capture the combined beam path 68. The image sensor is configured to resolve the combined beam path 68 according to the optical channels 30, 32. The image sensor features a Bayer filter (not shown here). The sensor unit 38 is connected to the computing unit 54 in terms of data technology. The computing unit 54 is configured to process the images captured by means of the sensor unit 38 for generating a stereoscopic image. The image sensor is arranged in such a way that the longitudinal axis 14 intersects the image sensor.

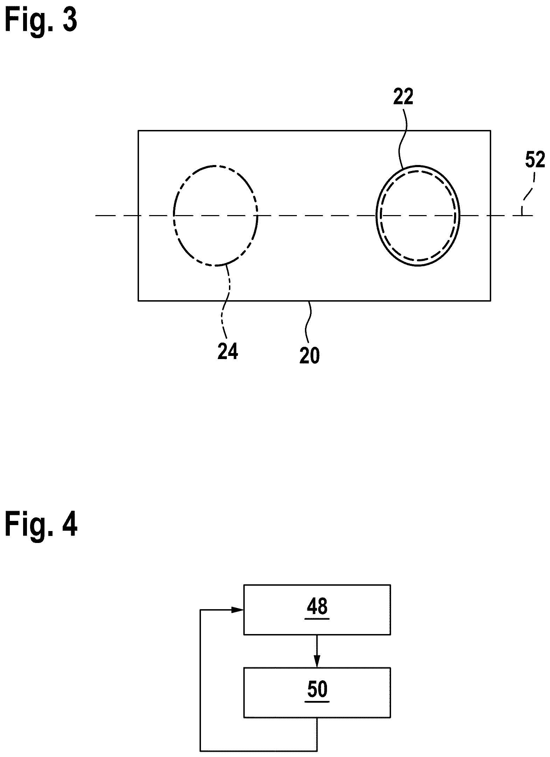

Fig. 4 shows a schematic sequence of a method for operating the imaging device 10.

In a method step, in particular in a capture step 48, images from different perspectives are transmitted via the beam paths 22, 24 to the sensor unit 38 via the two distinguishable optical channels 30, 32 and captured. The computing unit 54 generates a stereoscopic image from the images of the two optical channels 30, 32 and outputs it on the display 56.

In a method step, in particular in a rotation step 50, a viewing direction 62 of the imaging device 10 is changed by a rotation of the shaft 12, in particular of the viewing direction unit 58, about the longitudinal axis 14. The viewing direction 62 is defined by the viewing angle 18 and a rotation angle of the shaft 12, in particular the viewing direction unit 58, about the longitudinal axis 14. In the rotation step 50, the periscope unit 20 is rotated about the longitudinal axis 14 relative to the shaft 12. The periscope unit 20 is rotated in such a way that a plane 52 of the two beam paths 22, 24 running through the periscope unit 20 is aligned at least approximately horizontally (see Fig. 3).

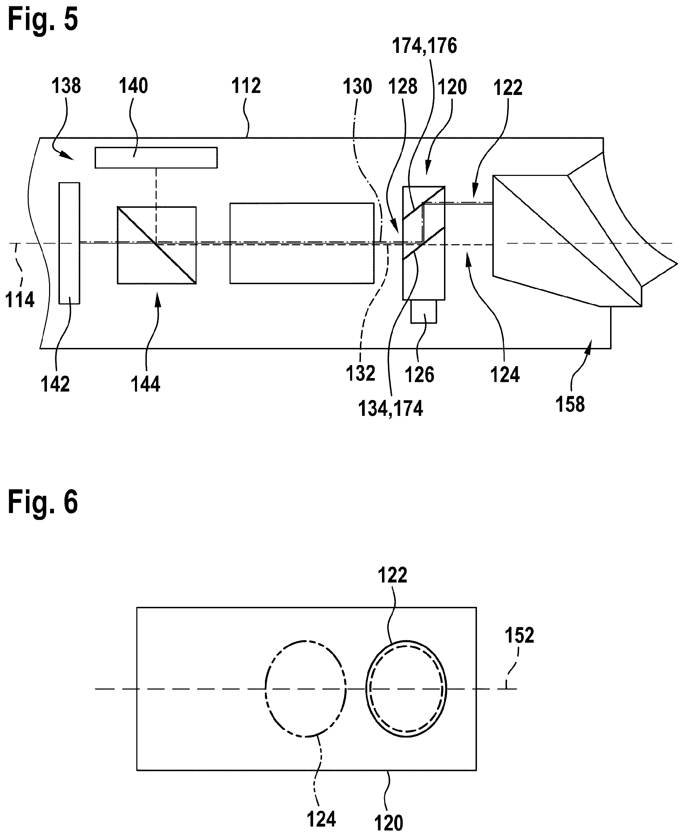

Fig. 5 shows an optical setup of an imaging device 110 for stereoscopic image generation in a further embodiment. The imaging device 110 has a shaft 112 that defines a longitudinal axis 114. The imaging device has at least one viewing direction unit 158 for generating a viewing angle greater than 0° with respect to the longitudinal axis 114. The imaging device 110 has a periscope unit 120 for combining two beam paths 122, 124 for use in stereoscopic image generation. The beam paths 122, 124 exit on a proximal side of the viewing direction unit 158. The beam path in the viewing direction unit 158 and on a distal side of the viewing direction unit 158 is not shown.

The viewing direction unit 158 is arranged in a rotationally fixed manner with respect to the shaft 112. The periscope unit 120 is arranged so as to be rotatable about the longitudinal axis 114 relative to the shaft 112. The periscope unit 120 has two distinguishable optical channels 130, 132 at its optical output 128.

The periscope unit 120 has a filter 134 in order to specify a beam characteristic with respect to at least one of the optical channels 130, 132. The periscope unit 120 deflects only a first beam path 122 of the two beam paths 122 and 124, wherein the periscope unit 120 has only two deflection elements 174. A first deflecting element 174 is designed as a mirror 176 and is arranged in a first beam path 122. A second deflection element 174 is formed by the filter 134. The filter 134 is designed as a dichroic beam splitter. Both beam paths 122, 124 interact with the filter 134. The filter 134 is designed to be transparent to the second optical channel 132.

The first beam path 122 runs at least distally with respect to the periscope unit 120, spaced apart from the longitudinal axis 114. The second beam path 124 runs along the longitudinal axis 114. The periscope unit 120 deflects the first beam path 122, so that the two beam paths 122, 124 are combined into a common beam path.

In the distal front view of the periscope unit 120 shown in Fig. 5, the two beam paths 122 and 124, which are spaced apart from one another, are shown. These span a plane 152, which is kept at least approximately horizontal by a rotation of the periscope unit 120 about the longitudinal axis 114 in the opposite direction to a rotation of the shaft 112 about the longitudinal axis 114 (see Fig. 6). The rotation of the periscope unit 120 can be generated by a movement unit 126.

The imaging device 110 has a sensor unit 138 for capturing the two beam paths 122, 124. The imaging device 110 has a beam splitter 144 located upstream of the sensor unit 138, which divides the combined beam paths 122, 124 according to their distinguishable optical channels 130, 132. The beam splitter 144 is designed as a dichroic beam splitter. The beam splitter 144 is configured to deflect the two optical channels 130, 132 in different directions. The beam splitter 144 is designed to be transparent to the first optical channel 132.

The sensor unit 138 has a dedicated image sensor 140, 142 in each case for the beam paths 122, 124, which are divided according to distinguishable optical channels 130, 132. The image sensors 140, 142 are designed as CMOS, CCD sensors or the like.

The configuration of the periscope unit 120 can also be combined with a single image sensor that is capable of resolving according to the optical channels 130 and 132. Furthermore, the periscope unit 20 can also be combined with the beam splitter 144 and the two image sensors 140, 142.

### List of reference signs

- 10, 110: Imaging device
- 12, 112: Shaft
- 14, 114: Longitudinal axis
- 16: Prism
- 18: Viewing angle
- 20, 120: Periscope unit
- 22, 122: Beam path
- 24, 124: Beam path
- 26, 126: Movement unit
- 28, 128: Optical output
- 30, 130: Optical channel
- 32, 132: Optical channel
- 34, 134: Filter
- 36: Filter
- 38, 138: Sensor unit
- 40, 140: Image sensor
- 142: Image sensor
- 144: Beam splitter
- 46: Imaging system
- 48: Capture step
- 50: Rotation step
- 52, 152: Plane
- 54: Computing unit
- 56: Display
- 58, 158: Viewing direction unit
- 60: Prism
- 62: Viewing direction
- 64: Distal end
- 66: Lens
- 68: Beam path
- 70: Optical system
- 72: Proximal end
- 74, 174: Deflection element
- 76, 176: Mirror
- 78: Dichroic beam splitter
- 80: Optical input

## Claims

1. Imaging device (10; 110), in particular an endoscope device, for stereoscopic image generation, comprising:
a shaft (12; 112) that defines a longitudinal axis (14; 114),
at least one optical viewing direction unit (58, 158) for generating a viewing angle (18) of greater than 0° with respect to the longitudinal axis (14; 114) and a periscope unit (20; 120) for combining at least two beam paths (22, 24; 122, 124) for use in stereoscopic image generation,
wherein the viewing direction unit (58, 158) is rotationally fixed with respect to the shaft (12; 112), and the periscope unit (20; 120) is arranged so as to be rotatable relative to the shaft (12; 112) about the longitudinal axis (14; 114).

2. Imaging device (10; 110) according to claim 1,
further comprising a movement unit (26; 126) that is configured to keep the periscope unit (20; 120) in a horizontal position when the shaft (12; 112) is rotated about the longitudinal axis (14; 114).

3. Imaging device (10, 110) according to claim 1 or 2,
wherein the periscope unit (20; 120) provides at least two distinguishable optical channels (30, 32; 130, 132) at its optical output (28; 128).

4. Imaging device (10, 110) according to claim 3,
wherein the optical channels (30, 32; 130, 132) have different color channels and/or different polarization channels.

5. Imaging device (10, 110) according to claim 3 or 4,
wherein the periscope unit (20; 120) has at least one filter (34, 36; 134) in order to specify a beam characteristic with respect to at least one of the optical channels (30, 32; 130).

6. Imaging device (10, 110) according to claim 5,
wherein the filter (34, 36; 134) is designed as a dichroic beam splitter.

7. Imaging device (10, 110) according to either claim 5 or claim 6,
wherein the filter (34, 36; 134) is designed to be transparent for one of the two optical channels (30, 32; 130, 132).

8. Imaging device (110) according to one of the preceding claims,
wherein the periscope unit (120) deflects only one of the two beam paths (122, 124).

9. Imaging device (10, 110) according to one of claims 3 to 7,
further comprising a sensor unit (38; 138) for capturing the two optical channels (30, 32; 130, 132).

10. Imaging device (10) according to claim 9,
wherein the sensor unit (38) has a single image sensor (40), in particular having a Bayer filter.

11. Imaging device (110) according to claim 9,
having a beam splitter (144) located upstream of the sensor unit (138), which divides the combined beam paths (122, 124) according to their distinguishable optical channels (130, 132).

12. Imaging device (110) according to claim 11, wherein the sensor unit (138) has a dedicated image sensor (140, 142) in each case for the beam paths (122, 124) divided according to distinguishable optical channels (130, 132).

13. Imaging system (46), in particular an endoscopy system, having an imaging device (10; 110) according to one of the preceding claims.

14. Method for operating an imaging device (10; 110) for stereoscopic image generation, in particular according to one of claims 1 to 12, comprising:
a shaft (12; 112) that defines a longitudinal axis (14; 114),
at least one viewing direction unit (58, 158) for generating a viewing angle (18) of greater than 0° with respect to the longitudinal axis (14; 114) and
a periscope unit (20; 120) for combining at least two beam paths (22, 24; 122, 124) for use in stereoscopic image generation,
wherein the viewing direction unit (58; 158) is arranged in a rotationally fixed manner with respect to the shaft (12; 112),
wherein the periscope unit (20; 120) is rotated relative to the shaft (12; 112) when the shaft (12; 112) is rotated about the longitudinal axis (14; 114).

15. Method for operating an imaging device (10; 110) for stereoscopic image generation according to claim 14,
wherein the periscope unit (20; 120) is rotated in such a way that a plane (52; 152) of the two beam paths (22, 24; 122, 124) running through the periscope unit (20; 120) is aligned at least approximately horizontally.
